# EUROPEAN PATENT SPECIFICATION

(11) **EP 2 051 688 B1**
(45) Date of publication and mention of the grant of the patent: **26.01.2011**
(21) Application number: 07787369.3
(22) Date of filing: 11.07.2007
(51) Int. Cl.: A61K 8/26, A61K 8/81, A61K 8/73, A61Q 5/06

(54) **HAIR TREATMENT COMPOSITION**
HAARBEHANDLUNGSZUSAMMENSETZUNG
COMPOSITION POUR LE TRAITEMENT DES CHEVEUX

(30) Priority: 16.08.2006 EP 06118978
(43) Date of publication of application: 29.04.2009
(73) Proprietor: Unilever PLC, London EC4Y 0DY (GB); Unilever N.V., 3013 AL Rotterdam (NL)
(72) Inventor: GILES, Colin, Christopher, David, Ladyao (TH); TREESILVATTANAKUL, Krissana, Ladyao (TH)
(74) Representative: Tansley, Sally Elizabeth
(86) International application number: PCT/EP2007/057095
(87) International publication number: WO 2008/019917

(56) References cited:
- EP-A2- 1 502 577
- WO-A-03/047541
- WO-A2-01/74311
- DE-A1- 19 847 808
- US-A- 5 112 603
- US-A1- 2004 229 991
- "STABILIZED SHAMPOO/SURFACTANT SUSPENSIONS" RESEARCH DISCLOSURE, MASON PUBLICATIONS, HAMPSHIRE, GB, vol. 434, June 2000 (2000-06), pages 1032-1033,AN434, XP009000038 ISSN: 0374-4353

## Description

### TECHNICAL FIELD

The present invention relates to a hair spray composition for styling and refreshing the hair.

### BACKGROUND OF THE INVENTION

Compositions on the market usually fall into two market segments. Rinse off products that are primarily for cleaning and/or conditioning the hair and leave-in products that are primarily for styling and/or conditioning hair.

WO 03/047541 (Unilever) discloses rinse off hair treatment compositions comprising particles of clay and charged organic molecules. The advantage of such compositions is that they leave the hair feeling smooth and moisturised.

JP05/246,824 (Shiseido Co Ltd) discloses a hair cosmetic which is a water-in-oil emulsion containing an organically modified clay mineral (prepared by treating a water swelling clay with a nonionic surfactant and a cationic surfactant), a water-soluble high molecular weight substance and a high molecular weight silicone. The benefits claimed are smooth feel, lustre, firmness and flexibility to hair.

The present invention recognises a need for a leave in product that can be used to both refresh and style the hair. To this effect, it has been found that a hair spray composition can be formulated that effectively styles the hair and leaves the hair feeling clean and smelling fresh. The product has the additional advantage that it can be used on wet or dry hair.

### DEFINITION OF THE INVENTION

According to a first aspect of the invention, there is provided a hair spray composition characterised in that it comprises a dispersion of
i) an anionic clay with a net negative surface charge; and
ii) a cationic styling polymer according to claim 1.

According to a second aspect of the invention, there is provided a method for styling hair and for modifying the odour of hair by applying to the hair the composition described above.

According to a third aspect of the invention, there is provided the use of a hair treatment composition as described above for styling hair and to freshen the hair.

### DETAILED DESCRIPTION OF THE INVENTION

### The clay

The term clay refers to a composition comprising fine particles which in this instance have a net negative electrostatic charge on at least one surface. Preferably, the clay has a layered structure comprising sheets of coordinated cations, wherein the cations form tetrahedra or octahedra with close packed oxygens and hydroxyl groups.

The surface charge is usually balanced by the presence of charge balancing ions (sometimes called exchangeable ions) which are usually present between the layers of the clay and at the edges of the layers.

The term anionic clays and related terms, as used herein, refer to clays which are negatively charged at their layer surface. In this embodiment, the composite particles are formed with a cationic charged organic molecule, which is in addition to and separate from any charge balancing cations which may be present.

The clay may be a natural, synthetic or chemically modified clay. Preferably, the layers of the clay comprise hydrous sheets of octahedrally coordinated aluminium, magnesium or iron, or of tetrahedrally coordinated silicon. The arrangement of octahedrally coordinated cations to coordinating anions in the clay sheets may be dioctahedral or trioctahedral. The charge balancing ions are cations at the layer surfaces and anions at the edges and may be those occurring naturally in the clay or by exchange using ion exchange techniques. The charge balancing cations of the anionic clays employed in the composite particles of the invention will contain cationic counterions such as protons, sodium ions, potassium ions, calcium ions, magnesium ions, and the like.

Preferred anionic clays are 2:1 phyllosilicates, in which the clay layers comprise two tetrahedral sheets to one octahedral sheet. Preferred 2:1 phyllosilicates are smectite clays.

Other suitable clays have a 1:1 phyllosilicate structure, with an assemblage of one tetrahedral to one octahedral coordinated sheet in the clay layers.

Smectite clays, used in laundry compositions are, for example, disclosed in US Patents Nos 3,862,058, 3,948,790, 3,954,632 and 4,062,647 and in EP-A-299, 575 and EP-A-3133,146, all in the name of Procter & Gamble Company.

The term smectite clays herein includes both the clays in which aluminium oxide is present in a silicate lattice and the clays in which magnesium oxide is present in a silicate lattice. Typical smectite clay compounds include the compounds having the general formula Al₂(Si₂O₅)₂(OH)₂.nH₂O and the compounds having the general formula Mg₃(Si₂O₅)₂(OH)₂·nH₂O, and derivatives thereof, for example in which a proportion of the aluminium ions are replaced with magnesium ions or a proportion of the magnesium ions are replaced with lithium ions and/or some of the hydroxyl ions are replaced by fluoride ions; the derivatives may comprise a further metal ion to balance the overall charge.

Specific examples of suitable smectite clays include those selected from the classes of the montmorillonites, hectorites, volchonskoites, nontronites, saponites, beidelites and sauconites, particularly those having an alkali or alkaline earth metal ion within the crystal lattice structure. Particularly preferred are hectorites, montmorillonites, nontronites, saponites, beidelites, sauconites and mixtures thereof. More preferably the clay is a synthetic hectorite.

The cation exchange capacity (CEC) of a clay is a well known parameter and may be determined by well established analytical techniques, including by electrodialysis, by exchange with ammonium ion followed by titration or by a methylene blue procedure, all as fully described in Grimshaw, "The Chemistry and Physics of Clays", pp. 264-265, Interscience (1971). It is customary to measure the cation exchange capacity of a clay in terms of milliequivalents per 100 g of dry clay (meq/100 g).

Preferred anionic clays for use in the present invention have a cation exchange capacity of from 0.7 meq/100 g to 150 meq/100 g. Particularly preferred are clays having a cation exchange capacity of from 30 meq/100 g to 100 meq/100 g.

The clays preferably have a volume-based median particle diameter (D_{0.5}) from 0.001 µm to 80 µm, more preferably from 0.01 µm to 50 µm and most preferably 0.02 µm to 20 µm. Particle diameters can be determined using a Malvern Mastersizer (Malvern Instruments, UK).

Examples of synthetic hectorites useful in the present invention include those products sold under the trade names Laponite RD, Laponite RDS, Laponite XLG, Laponite XLS, Laponite D, Laponite DF, Laponite DS, Laponite S and Laponite JS (all from Southern Clay products, Texas, USA, a subsidiary of Rockwood).

Examples of montmorillonites (also known as bentonites), which are suitable for use in the present invention include: Gelwhite GP, Gelwhite H, Gelwhite L, Mineral Colloid BP, Mineral Colloid MO, Gelwhite MAS 100 (sc), Gelwhite MAS 101, Gelwhite MAS 102, Gelwhite MAS 103, Bentolite WH, Bentolite L10, Bentolite H, Bentolite L, Permont SX10A, Permont SC20, and Permont HN24 (Southern Clay Products, Texas, USA); Bentone EW and Bentone MA (Dow Corning); Bentonite USP BL 670 and Bentolite H4430 (Whitaker, Clarke & Daniels); Clarit 100 G1 and Clarit 1100 G1 (calcium bentonites from Süd Chemie AG); and Volclay 2 (sodium bentonite from Süd Chemie AG) .

Clays may be used as obtained from the supplier and may contain conventional additives such as, for example, disintegrating agents (also known as peptisers) and water of hydration. The clays may be used in their natural state or in a purified or semi-purified form, for example with the removal of mineral impurities.

The level of the clay in the total composition is preferably from 0.01 to 10 wt% of the total composition, more preferably from 0.02 to 5 wt%, most preferably from 0.05 to 2 wt%.

In the compositions of the invention, the clay is advantageously present in the form of a dispersion (for example a sol or gel) or as a suspension.

### Cationic Styling Polymer

These compositions of the invention comprise at least one hair styling polymer according to claim 1.

Hair styling polymers are well known articles of commerce and styling such polymers are available commercially which contain moieties which render the polymers cationic in nature .The polymers may be synthetic or naturally derived.

The amount of the polymer may range from 0.01 to 10%, more preferably 0.05 to 6%, most preferably from 0.1 to 4 % by weight the total composition.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as H-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat®FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally derived polymers include shellac, alginates, gelatins, pectins, starch, cellulose derivatives and chitosan or salts and derivatives thereof. Commercially available examples include Kytamer® (ex Amerchol) and Amaze® (ex National Starch).

The weight ratio of the clay to the cationic styling polymer is from 1:5 to to 5:1, more preferably 1:3 to 3:1, most preferably from 1:1 to 3:1.

### Silicone Conditioning Agents

The compositions of the invention preferably contain silicone conditioning agents selected from volatile and non-volatile silicone fluids.

Silicones may be added as a pre-formed emulsion. Viscosities of the pre-emulsified silicones range up to 10McP at 25°C

Volatile silicone fluids are preferably oils chosen from cyclic or linear polydimethyl siloxanes containing from 3 to 9, preferably from 4 to 5 silicon atoms.

Cyclomethicone is the most preferred cyclic volatile silicone. Linear volatile silicone oils generally have viscosities less than about 5 centistokes at 25°C while cyclic fluids typically have viscosities of less than about 10 centistokes.

Non-volatile silicone oils useful for the present invention include polyalkyl siloxanes, polyalkylaryl siloxanes and polyether siloxane copolymers. Non-volatile polyalkyl siloxanes useful herein include, for example, polydimethyl siloxanes with viscosities of from 5 to 100,000 centistokes at 25°C. Among the preferred non-volatile silicones are the polydimethyl siloxanes having viscosities from 10 to 400 centistokes at 25°C. These siloxanes are available, for example, from the General Electric Company as SF 1075 methyl phenyl fluid or from Dow Corning as 556 Cosmetic Grade Fluid.

The non-volatile polyalkylaryl siloxane fluids that may be used include, for example, polymethylphenylsiloxanes having viscosities of about 15 to 30,000 centistokes at 25°C.

### Further Components

The aqueous hair styling aids of the invention can contain a variety of nonessential, optional components suitable for rendering the compositions more aesthetically acceptable or to aid use, including discharge from the container, of the product. Such conventional optional ingredients are well known to those skilled in the art, e.g.surfactants, particularly preferred are non-ionic surfactants, preservatives such as benzyl alcohol, methyl paraben, propyl paraben and imidazolidinyl urea, fatty alcohols such as cetearyl alcohol, cetyl alcohol and stearyl alcohol, pH adjusting agents such as citric acid, succinic acid, sodium hydroxide and triethanolamine, colouring agents such as any of the FD&C or D&C dyes, perfume oils, chelating agents such as ethylenediamine tetraacetic acid, polymer plasticising agents such as glycerin and propylene glycol.

### Hair Treatment Compositions

Compositions in accordance with the invention are preferably formulated as leave on products for the treatment of hair without subsequent rinsing.

It is preferred if the compositions are delivered in the form of a spray, particularly preferred is an aqueous/ethanolic spray. The preferred level of ethanol in the composition is from 3 to 50wt% of the total composition, more preferably from 5 to 20 wt%.

### EXAMPLES

The invention will now be further illustrated by the following, non-limiting Examples. Examples of the invention are illustrated with a number, comparative Example with a letter.

### Example A

| Chemical Name | Trade Name | % active |
|---|---|---|
| Polyoxyl 40 hydrogenated | Cremophore RH40 ⁽¹⁾ | 1.00% |
| Castor oil | | |
| Dimethicone/cyclomethicone | DC1787 ⁽²⁾ | 0.5% |
| Ethanol | | 10.0% |
| Polyquaternium 16 | Luviquat FC550 ⁽³⁾ | 0.3% |
| Preservatives, water & minors | | to 100% |

### Preparation

The main vessel is charged with 40.3% of the water. In a side mixed the fragrance and Cremophore are blended before adding into the main vessel, additional water is used to flush the side vessel. The main vessel is stirred at moderate speed until homogeneous. In the side vessel a solution of the styling polymer in water (20%) is prepared, the preservatives are added. This premix is stirred until homogene is and is then dispelled into the main vessel, followed by an additional flushing with water. The main vessel in again stirred under moderate shear until homogeneous. In the side vessel the silicone emulsion is diluted with water and this mixture is added to the main vessel. The main vessel is then stirred until homogeneous and the pH adjusted before discharging.

### Example 1

| Chemical Name | Trade Name | %Active |
|---|---|---|
| Polyoxyl 40 hydrogenated Castor oil | Cremophor RH40 | 1.00% |
| Dimethicone/cyclomethicone | DC17877⁽¹⁾ | 0.5% |
| Ethanol | | 10.0% |
| Polyquaternium 16 | Luviquat FC550 ⁽²⁾ | 0.3% |
| Synthetic silicate | Laponite XLS⁽³⁾ | 0.3% |
| Preservatives, water & minors | | to 100% |

### Preparation

The main vessel is charged with 40% of the water. The Laponite is added slowly with agitation to the main vessel, stirring is continued until the clay is fully dispersed in the water. In a side mixer the fragrance and Cremophore are blended before adding into the main vessel, additional water is used to flush the side vessel. The main vessel is stirred at moderate speed until homogeneous. In the side vessel a solution of the styling polymer in water (20%) is prepared, the preservatives are added. This premix is stirred until homogeneous and is then dispelled into the main vessel, followed by an additional flushing with water. The main vessel is again stirred under moderate shear until homogeneous. In the side vessel the silicone emulsion is diluted with water and this mixture is added to the main vessel. The main vessel is then stirred until homogeneous and the pH adjusted before discharging.

### Example 2

| Chemical Name | Trade Name | % Active |
|---|---|---|
| Polyoxyl 40 Hydrogenated Castor oil | Cremophor RH40 | 1.00% |
| Dimethicone/cyclomethicone | DC1787 ⁽¹⁾ | 0.5% |
| Ethanol | | 10.0% |
| Polyquaternium 16 | Luviquat FC550 ⁽²⁾ | 0.3% |
| Synthetic silicate | Laponite XLS ⁽³⁾ | 0.45% |
| Preservatives, water & minors | | to 100% |

| | | |
|---|---|---|
| ⁽¹⁾ supplies by Dow Corning ⁽²⁾ supplied by BASF ⁽³⁾ supplied by Rockwood | | |

Example 2 is prepared in the same manner as example 1, the only difference is that the clay level is increased to 0.45%

### Evaluation 1

### Methodology

36 women with normal and normal to greasy hair were selected for this test. The respondents washed their hair with a control shampoo 24 hours prior to application of the product. The respondents were treated with both the comparative Example A and example 1 in a random half head design. Four sprays of both products were applied to each half of the respondent's head by a trained stylist.

The respondent assessed the product during application and then they are allowed to style their own hair using whatever tools they prefer. The respondent and stylist assessed each half of the respondent's head in order to determine which product performed better on which attribute.

### Results

The results for the evaluation are shown below, only significant differences (student t-test) are illustrated, with the level of significance highlighted in brackets

| Stylist N=36 | Total respondent n=36 | Normal to greasy respondents n=19 |
|---|---|---|
| Hold (99%) | Ease of Style (90%) | Scalp freshness (90%) |
| Flyaway (90%) | Shininess (95%) | Ease of Style (95%) |
| | | Best hair style (90%) |
| | | Shininess (90%) |
| | | Pleasant smell (90%) |

### Evaluation 2

### Methodology

The Examples were tested as described above.

### Results

The results are illustrated below. The results demonstrate the performance of the invention when applied to either damp or dry hair.

| **Example A vs Example 1** | | **Example 1 vs Example 2** | |
|---|---|---|---|
| **Example 1 preferred (Sig).** | | **Example 2 preferred (Sig)** | |
| Applied on wet | Applied on dry | Applied on wet | Applied on dry |
| Less frizz (90%) | Ease of combing (90%) | | Moisturisation @ application (95%) |
| Less coating (95%) | Soft feel (95%) | | Soft @ application (95%) |
| | Clean feel (90%) | | Smooth @ application (95%) |
| | Shine (95%) | | Less Greasy/oily (95%) |
| | Less dryness (95%) | | Fresh feel (95%) |
| | Fresh Next day (95%) | | Fresh look (95%) |
| | | | Shine (95%) |

It will be seen that feel attributes are significantly increased on hair treated with compositions according to the invention, compared to hair treated with Comparative Example A.

## Claims

1. A hair spray composition **characterised in that** it comprises a dispersion of
i) a clay with a net negative surface charge, and
ii) a cationic styling polymer, selected from the group consisting of copolymers of amino-functional acrylate , copolymers of N-vinyl pyrrolidone and dimethylaminoethyl methacrylate, copolymers of N-vinyl pyrrolidone, vinylcaprolactam and dimethylamino ethylmethacrylate, N-vinylpyxrolidone and dimethylaminopropylacrylamide or methacrylamide, polyquaternium-4, polyquaterium-11, polyquaternium-16 and polyquaternium-46 in which the weight ratio of the clay to the cationic styling polymer is from 1:5 to 5:1.

2. A composition according to claim 1 in which the weight ratio of the clay to the catienic styling polymer is from 1:1 to 3:1.

3. A composition according to claim 1 or claim 2 in which the clay is a synthetic hectorite.

4. A composition as claimed in any preceding claim in which the level of clay is from 0.05 to 2 wt% of the total composition.

5. A composition according to any preceding claim in which the cationic styling polymer comprises a methylvinylimidazolium copolymer.

6. A composition according to claim 5 in which the methylvinylimidazoline is Polyquaternium 16.

7. A composition according to any preceding claim in which the level of cationic styling polymer is from 0.1 to 4 wt% of the total composition.

8. A composition according to any preceding claim that further comprises a silicone.

9. A method for styling hair **characterised in that** it comprises applying to the hair a composition as claimed in any one of claims 1 to 8.

10. A method for modifying the odour of hair, **characterised in that** it comprises applying to the hair a composition as claimed in any one of claims 1 to 8.

11. Use of a hair treatment composition as claimed in any one of claims 1 to 8 for styling hair.

## Patentansprüche

1. Haarsprühzusammensetzung, **dadurch gekennzeichnet, dass** sie eine Dispersion von Folgendem aufweist:
i) Tonerde mit einer insgesamt negativen Oberflächenladung und
ii) ein kationisches Formgebungspolymer, das aus der Gruppe ausgewählt ist, bestehend aus Copolymeren von Amino-funktionellem Acrylat, Copolymeren von N-Vinylpyrrolidon und Dimethylaminoethylmethacrylat, Copolymeren von N-Vinylpyrrolidon, Vinylcaprolactam und Dimethylaminoethylmethacrylat, N-Vinylpyrrolidon und Dimethylaminopropylacrylamid oder -methacrylamid, Polyquaternium-4, Polyquaternium-11, Polyquaternium-16 und Polyquaternium-46, wobei das Gewichtsverhältnis zwischen der Tonerde und dem kationischen Formgebungspolymer 1:5 bis 5:1 beträgt.

2. Zusammensetzung nach Anspruch 1,
wobei das Gewichtsverhältnis zwischen der Tonerde und dem kationischen Formgebungspolymer 1:1 bis 3:1 beträgt.

3. Zusammensetzung nach Anspruch 1 oder 2,
wobei die Tonerde synthetisches Hectorit ist.

4. Zusammensetzung nach einem der vorstehenden Ansprüche,
wobei die Menge der Tonerde 0,05 bis 2 Gew.-% der gesamten Zusammensetzung beträgt.

5. Zusammensetzung nach einem der vorstehenden Ansprüche,
wobei das kationisches Formgebungspolymer ein Methylvinylimidazolium-Copolymer aufweist.

6. Zusammensetzung nach Anspruch 5,
wobei das Methylvinylimidazolin Polyquaternium-16 ist.

7. Zusammensetzung nach einem der vorstehenden Ansprüche,
wobei die Menge des kationischen Formgebungspolymers 0,1 bis 4 Gew.-% der gesamten Zusammensetzung beträgt.

8. Zusammensetzung nach einem der vorstehenden Ansprüche,
die ferner ein Silicon aufweist.

9. Formgebungsverfahren für Haare, **dadurch gekennzeichnet, dass**
es das Aufbringen einer Zusammensetzung nach einem der Ansprüche 1 bis 8 auf das Haar aufweist.

10. Verfahren zum Modifizieren des Geruchs von Haaren, **dadurch gekennzeichnet, dass** es das Aufbringen einer Zusammensetzung nach einem der Ansprüche 1 bis 8 auf das Haar aufweist.

11. Verwendung einer Haarbehandlungszusammensetzung nach einem der Ansprüche 1 bis 8 für die Formgebung von Haaren.

## Revendications

1. Composition de laque capillaire **caractérisée en ce qu'**elle comprend une dispersion de
i) une argile possédant une charge superficielle négative nette, et
ii) un polymère fixateur cationique choisi dans le groupe constitué par les copolymères de monomères d'acrylate à fonctionnalité amino, les copolymères de N-vinylpyrrolidone et de méthacrylate de diméthylaminoéthyle, les copolymères de N-vinylpyrrolidone, de vinylcaprolactame et de méthacrylate de diméthylaminoéthyle, les copolymères de N-vinylpyrrolidone et de diméthylaminopropylacrylamide ou de méthacrylamide, le polyquaternium-4, le polyquaternium-11, le polyquaternium-16 et le polyquaternium-46,
dans laquelle le rapport en poids de l'argile au polymère fixateur cationique est compris dans la plage allant de 1/5 à 5/1.

2. Composition selon la revendication 1, dans laquelle le rapport en poids de l'argile au polymère fixateur cationique est compris dans la plage allant de 1/1 à 3/1.

3. Composition selon la revendication 1 ou la revendication 2, dans laquelle l'argile est une hectorite synthétique.

4. Composition selon l'une quelconque des revendications précédentes, dans laquelle le taux d'argile est compris dans la plage allant de 0,05 à 2 % en poids de la composition totale.

5. Composition selon l'une quelconque des revendications précédentes, dans laquelle le polymère fixateur cationique comprend un copolymère de méthylvinylimidazolium.

6. Composition selon la revendication 5, dans lequel la méthylvinylimidazoline est le polyquaternium-16.

7. Composition selon l'une quelconque des revendications précédentes, dans laquelle le taux de polymère fixateur cationique est compris dans la plage allant de 0,1 à 4 % en poids de la composition totale.

8. Composition selon l'une quelconque des revendications précédentes, comprenant en outre une silicone.

9. Procédé de fixation des cheveux **caractérisé en ce qu'**il comprend l'application aux cheveux d'une composition selon l'une quelconque des revendications 1 à 8.

10. Procédé de modification de l'odeur des cheveux, **caractérisé en ce qu'**il comprend l'application aux cheveux d'une composition selon l'une quelconque des revendications 1 à 8.

11. Utilisation d'une composition de traitement capillaire selon l'une quelconque des revendications 1 à 8 pour la fixation des cheveux.
